(19) Europäisches Patentamt
European Patent Office
Office européen des brevets

(11) Publication number : 0 348 942 B1

(12) # EUROPEAN PATENT SPECIFICATION

(45) Date of publication of patent specification : **09.12.92 Bulletin 92/50**

(51) Int. Cl.$^5$ : **A61K 37/22,** A61K 9/10

(21) Application number : **89111775.6**

(22) Date of filing : **28.06.89**

(54) **Lipid membrane structures.**

(30) Priority : **29.06.88 JP 162153/88**

(43) Date of publication of application : **03.01.90 Bulletin 90/01**

(45) Publication of the grant of the patent : **09.12.92 Bulletin 92/50**

(84) Designated Contracting States : **CH DE FR GB IT LI NL SE**

(56) References cited :
**WO-A-87/04926**
**DE-A- 3 621 306**

(73) Proprietor : **DAIICHI PHARMACEUTICAL CO., LTD.**
**14-10, Nihonbashi 3-chome**
**Chuo-ku, Tokyo 103 (JP)**

(72) Inventor : **Tomikawa, Munehiro Daiichi Pharmaceutical**
**Research Institute 16-13, Kitakasai 1-chome**
**Edogawa-ku Tokyo (JP)**
Inventor : **Hirota, Sadao Daiichi Pharmaceutical**
**Research Institute 16-13, Kitakasai 1-chome**
**Edogawa-ku Tokyo (JP)**
Inventor : **Yamauchi, Hitoshi Daiichi Pharmaceutical**
**Research Institute 16-13, Kitakasai 1-chome**
**Edogawa-ku Tokyo (JP)**
Inventor : **Kikuchi, Hiroshi Daiichi Pharmaceutical**
**Research Institute 16-13, Kitakasai 1-chome**
**Edogawa-ku Tokyo (JP)**
Inventor : **Kawato, Yasuyoshi Daiichi Pharmaceutical**
**Research Institute 16-13, Kitakasai 1-chome**
**Edogawa-ku Tokyo (JP)**

(74) Representative : **Kinzebach, Werner, Dr. et al**
**Patentanwälte Reitstötter, Kinzebach und Partner Sternwartstrasse 4 Postfach 86 06 49**
**W-8000 München 86 (DE)**

EP 0 348 942 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

FIELD OF THE INVENTION

This invention relates to lipid membrane structures which are delivered preferentially to tumor cells, and are therefore useful as a drug carrier in medical treatment.

BACKGROUND OF THE INVENTION

In the studies of liposomes which are delivered preferentially to tumor cells, methods for modifying liposomal surfaces with monoclonal antibodies have hitherto been reported, e.g., in Tadakuma, Iyaku Journal, Vol. 20, p. 643 (1984), Tadakuma, Saibo Kogaku, Vol. 1, p. 72 (1982), Ohsawa et al., Chemical and Pharmaceutical Bulletin, Vol. 35, p. 740 (1987), and Papahadjopoulos et al., Cancer Research, Vol. 46, p. 4904 (1986). In particular, Ohsawa et al. reported that small unilamellar liposomes modified with anti-carcinoembryonic antigen antibodies were easily taken up by tumor cells to exhibit enhanced antitumor effects.

In the present situation, however, the monoclonal antibodies are difficult to commercialize because of the difficulty of mass-production.

One object of this invention is to provide lipid membrane structures which are delivered preferentially to tumor cells and can be mass-produced with good reproducibility.

As a result of extensive investigations, the inventors have found that the above object of this invention can be accomplished by lipid membrane structures containing, in the lipid membrane thereof, a compound represented by formula (I):

$$R_3-(CH_2)_n\underset{}{CH}(NHR_1)COOR_2 \qquad (I)$$

wherein $R_1$ represents a hydrogen atom or a fatty acid residue having 1 to 30 carbon atoms;
$R_2$ represents a hydrogen atom or an acyclic hydrocarbon residue having 1 to 30 carbon atoms, provided that $R_1$ and $R_2$ do not represent hydrogen atoms at the same time and that the total number of carbon atoms of $R_1$ and $R_2$ is 10 to 40;
$R_3$ represents an amino group, a guanidino group or an amidino group; and n represents an integer of from 1 to 6; or a salt thereof, wherein the molar ratio of said compound of formula (I) or a salt thereof to the total content of the lipid components is at least about 1/40.

In formula (I), the term "fatty acid residue" means a group derived from a saturated or unsaturated fatty acid which may have a branch by removing one hydroxyl group therefrom. Specific examples of the fatty acid residue include those having from 1 to 30 carbon atoms, preferably from 14 to 20 carbon atoms, e.g., formyl, acetyl, propanoyl, butanoyl, pentanoyl, hexanoyl, heptanoyl, octanoyl, decanoyl, dodecanoyl, tridecanoyl, tetradecanoyl, pentadecanoyl, hexadecanoyl, heptadecanoyl, octadecanoyl, nonadecanoyl, eicosanoyl, heneicosanoyl, docosanoyl, tricosanoyl, tetracosanoyl, hexacosanoyl, triacontanoyl, 9-hexadecenoyl, 9-octadecenoyl, 9,12-octadecadienoyl, 9,12,15-octadecatrienoyl, 11-eicosenoyl, 11,14-eicosadienoyl, 11,14,17-eicosatrienoyl, 4,8,12,16-eicosatetraenoyl, 13-docosenoyl, 4,8,12,15,19-docosapentaenoyl, 15-tetracosenoyl, 2-dodecylhexadecanoyl, 2-tetradecylhexadecanoyl, 2-dodecyltetradecanoyl, 2-tetradecenylhexadecenoyl, 2-tetradecylhexadecenoyl, 2-tetradecenylhexadecanoyl and 2-dodecyloctadecanoyl groups.

The term "acyclic hydrocarbon residue" means a group derived from a saturated or unsaturated acyclic hydrocarbon which may have a branch by removing one hydrogen atom therefrom. Specific examples of the acyclic hydrocarbon residue include those having from 1 to 30 carbon atoms, preferably from 14 to 20 carbon atoms, e.g., methyl, ethyl, propyl, butyl, pentyl, hexyl, heptyl, octyl, nonyl, decyl, dodecyl, tridecyl, tetra-decyl, pentadecyl, hexadecyl, heptadecyl, octadecyl, nonadecyl, eicosyl, heneicosyl, docosyl, tricosyl, tetracosyl, hexacosyl, triacontyl, 9-hexadecenyl, 9-octadecenyl, 9,12-octadecadienyl, 9,12,15-octadecatrienyl, 11-eicosenyl, 11,14-eicosadienoyl, 11,14,17-eicosatrienyl, 4,8,12,16-eicosatetraenyl, 13-docosenyl, 4,8,12,15,19-docosapentaenyl, 15-tetracosenyl, 2-dodecyltetradecyl, 2-dodecylhexadecyl, 2-tetradecylhexadecyl, 2-tetradecylhexadecenyl, 2-tetradecenylhexadecyl, and 2-dodecyloctadecyl groups.

"n" in formula (I) preferably represents 3 or 4.

Of the compounds represented by formula (I), preferred are those wherein $R_1$ is a fatty acid residue and $R_2$ is an acyclic hydrocarbon residue. In these preferred compounds, the total number of the carbon atoms of

$R_1$ and $R_2$ is preferably in the range of from 10 to 40.

The "lipid membrane structures" according to the present invention means lamellar lipid particles wherein polar head groups of a polar lipid are arranged to face an aqueous phase of an interface to form membrane structures. Examples of the lipid membrane structures include liposomes, micelles and microemulsions.

A process for preparing the lipid membrane structures containing the compound of formula (I) or a salt thereof in the lipid membrane thereof is described below.

(a) Preparation of Liposomes Containing Compound (I) or a Salt Thereof in Liposomal Membrane:

An aqueous dispersion of liposomes is prepared from membrane components, such as phospholipids (e.g., phosphatidylcholine, phosphatidylglycerol,sphingomyelin, and phosphatidylethanolamine), glycolipids, and dialkyl-type synthetic surfactants according to the known methods as disclosed, e.g., in Annual Review of Biophysics and Bioengineering, Vol. 9, p. 467 (1980). The liposomes may further contain sterols (e.g., cholesterol and cholestanol), dialkylphosphates, diacylphosphatidic acids, stearylamine or α-tocopherol, in the liposomal membrane.

To the liposomal dispersion thus prepared is added an aqueous solution of the compound of formula (I) or a salt thereof, and the mixture is allowed to stand for a given period of time, preferably under warming at a temperature more than the phase transition temperature of the membrane or above 40°C, followed by cooling to thereby prepare liposomes containing the compound of formula (I) or a salt thereof in the liposomal membrane.

Alternatively, the desired liposomes can also be prepared by previously mixing the above-described membrane components and the compound of formula (I) or a salt thereof, and treating the mixture in accordance with the known methods for preparing liposomes.

(b) Preparation of Micelles Containing Compound (I) or a Salt Thereof in Micellar Membrane:

A micelle-forming surfactant, such as polyoxyethylene sorbitan fatty acid esters, polyoxyethylene castor oil derivatives, polyoxyethylene hardened castor oil derivatives, fatty acid sodium salts, sodium cholates, polyoxyethylene fatty acid esters, and polyoxyethylene alkyl ethers, alkyl glycosides, is added to water at a concentration above the critical micelle concentration to prepare a micellar dispersion. To the micellar dispersion is added an aqueous solution of the compound of formula (I) or a salt thereof, and the mixture is allowed to stand for a given period of time, preferably under warming at 40°C or higher, followed by cooling to thereby prepare micelles containing the compound of formula (I) or a salt thereof in the micellar membrane. Alternatively, the desired micelles can also be prepared by previously mixing the above-described micelle-forming substances and the compound of formula (I) or a salt thereof and treating the mixture according to the known methods for micelles formation.

(c) Preparation of Microemulsions Containing Compound (I) or a Salt Thereof in Lipid Membrane Thereof:

To the micelles as prepared in (b) above are added fats and oils, such as soybean oil, to saturate the micelles with the fats and oils, and to increase the oily phase to such a degree that no irreversible oil phase separation may not occur, to thereby prepare microemulsions containing the compound of formula (I) or a salt thereof in the lipid membrane thereof. Alternatively, the desired microemulsions can also be prepared by adding an aqueous solution of the compound of formula (I) or a salt thereof to microemulsions previously prepared according to known methods, and the resulting emulsions are allowed to stand for a given period of time, preferably under warming at 40°C or higher, followed by cooling.

In some cases of the above-described processes, the form of the resulting lipid membrane structures may be varied by controlling the proportion of the compound of formula (I) or a salt thereof to the total content of the lipid components. For instance, in the case that phosphatidylcholine is used as a sole lipid component, liposomes can be produced when a molar ratio of the compound of formula (I) or a salt thereof to the total content of the lipid components is adjusted to about 2/3 or less; and micelles or microemulsions can be produced when the above-described molar ratio is greater than 2/3.

In order to deliver preferentially the lipid membrane structures according to the present invention to tumor cells, it is usually desirable to use the compound of formula (I) or a salt thereof at a molar ratio of at least about 1/40 to the total content of the lipid components.

The compound of formula (I) can be prepared by known methods, and typical processes for preparing the compound of formula, (I) are illustrated below.

(1) Preparation of Compound (I) [$R_3$: guanidino group]

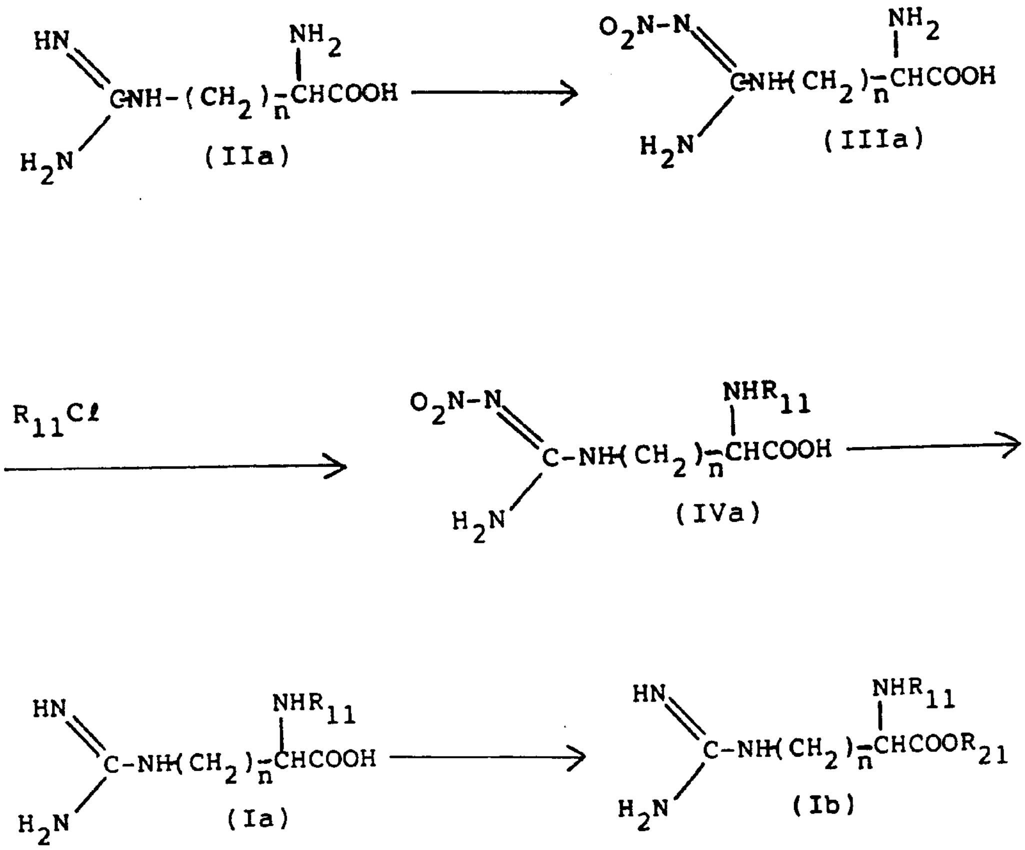


(IIa) (IIIa) (IVa) (Ia) (Ib)

wherein $R_{11}$ represents a fatty acid residue; $R_{21}$ represents an acyclic hydrocarbon residue; and n is as defined above.

The compound of formula (IIa) can be reacted with nitric acid in an appropriate organic solvent in the presence of sulfuric acid to prepare the compound of formula (IIIa). The compound of formula (IIIa) can be reacted with a fatty acid chloride ($R_{11}$ $C\ell$) in an appropriate organic solvent in the presence of a base, e.g. sodium hydroxide, to prepare the compound of formula (IVa), which can be then catalytically reduced in an appropriate organic solvent in the presence of a catalyst, e.g. palladium carbon, to prepare the desired compound of formula (Ia). The compound of formula (Ib) can then be obtained by esterification of the compound of formula (Ia) with a compound of formula $R_{21}OH$ in an appropriate organic solvent in the presence of an acid.

When the compound of formula (IIa) is reacted according to the esterification, a compound of formula (I) wherein $R_1$ is a hydrogen atom, $R_2$ is an acyclic hydrocarbon residue, and $R_3$ is a guanidino group can be obtained.

(2) Preparation of Compound (I) [$R_3$: amino group]

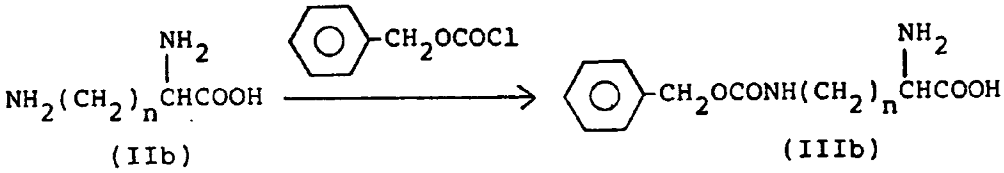

(IIb) (IIIb)

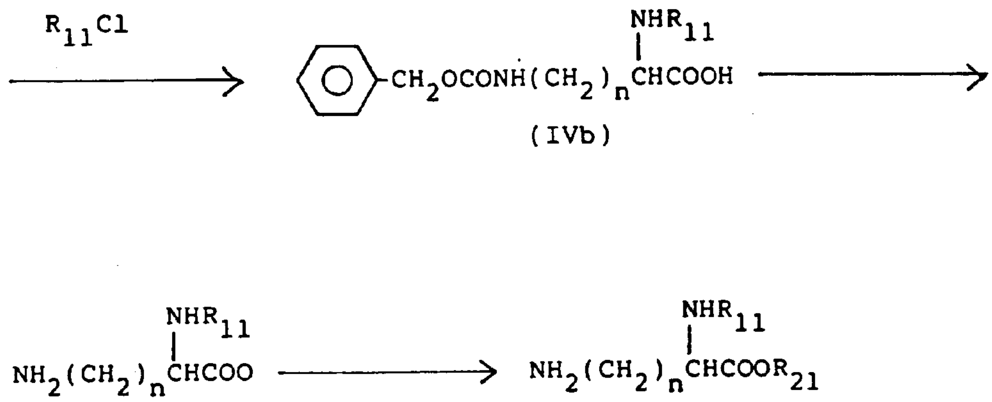


(IVb)

(Ic) (Id)

wherein $R_{11}$, $R_{21}$, and n are as defined above.

The compound of formula (IIb) can be reacted with benzyloxycarbonyl chloride in an appropriate organic solvent to prepare the compound of formula (IIIb). The compound of formula (IIIb) can be reacted with a fatty acid chloride $R_{11}C\ell$ in an appropriate organic solvent in the presence of an alkali, e.g. sodium hydroxide, to prepare the compound of formula (IVb). The compound of formula (IVb) can be catalytically reduced in an appropriate organic solvent in the presence of a catalyst, e.g. palladium carbon, to prepare the compound of formula (Ic). The compound of formula (Ic) can be then esterified with a compound of formula $R_{21}OH$ in an appropriate organic solvent in the presence of an acid to obtain the compound of formula (Id).

When the compound of formula (IIb) is reacted according to the esterification, a compound of formula (I) wherein $R_1$ is a hydrogen atom, $R_2$ is an acyclic hydrocarbon residue, and $R_3$ is an amino group can be produced.

The compound of formula (I) has optical isomers. These isomers and a mixture thereof are included in the scope of the present invention.

Drugs that may be encapsulated in the lipid membrane structures of the present invention vary depending on the type of the membrane structures. For example, the drugs that may be encapsulated in the liposomes are not particularly limited and include water-soluble drugs and lipid soluble drugs, such as Methotrexate and Cisplatin. The drugs which may be encapsulated in the micelles or microemulsions include lipid soluble drugs.

In the lipid membrane of the present invention, the compound of formula (I) or a salt thereof is firmly incorporated into the lamellar lipid membrane thereof through a hydrophobic interaction. It has been confirmed by gel filtration and Test Example 1 hereinafter given that the proportion of the compound of formula (I) or a salt thereof that exists as a free monomer to that in the lipid membrane is very low.

The lipid membrane structures according to the present invention possess excellent specific affinity for tumor cells, therefore, the lipid membrane structures of the present invention can be delivered preferentially to tumor cells. Further, the compound of formula (I) and salts thereof can be produced chemically on a large scale, therefore, the lipid membrane structures of the present invention can be prepared in large quantity with good reproducibility.

The present invention is now illustrated in greater detail by way of the following Examples and Test Examples.

COMPARATIVE EXAMPLE 1

Dipalmitoylphosphatidylcholine (hereinafter abbreviated as DPPC) and cholesterol were put in a test tube at a molar ratio of 1:1 to a total lipid amount of 8 μmol and dissolved in chloroform. The chloroform was then removed in a nitrogen gas stream to form a lipid film on the inner wall of the tube. Two milliliters of a phosphate-buffered saline (pH = 7.4, hereinafter abbreviated as PBS) were added thereto. After shaking in a vortex mixer, the mixture was subjected to sonication to prepare a liposomal dispersion. The dispersion was warmed to 45 to 50°C and then passed through a polycarbonate membrane filter having a pore size of 0.2 μm to prepare a liposomal dispersion having a particle size of not greater than 0.2 μm. The dispersion was subjected to ultracentrifugation (150,000 × g, 1 hour, twice), the supernatant was removed, and 5 ml of PBS was added to obtain a liposomal dispersion.

EXAMPLE 1

DPPC, cholesterol, and $N^{\alpha}$-cocoyl-L-arginine ethyl ester (hereinafter abbreviated as CAEE) were put in a test tube at a molar ratio of 1:1:0.05 to a total lipid amount of 8 μmol and dissolved in a 9:1 (by volume) mixture of chloroform and methanol. The solution was then treated in a similar manner to that in Comparative Example 1 to prepare a liposomal dispersion.

EXAMPLE 2

DPPC, cholesterol, and CAEE were put in a test tube at a molar ratio of 1:1:0.1 to a total lipid amount of 8 μmol and then the solution was treated in a similar manner to that in Comparative Example 1 to prepare a liposomal dispersion.

EXAMPLE 3

DPPC, cholesterol, and CAEE were put in a test tube at a molar ratio of 1:1:0.15 to a total lipid amount of 8 μmol and dissolved in a 9:1 (by volume) mixture of chloroform and methanol. The solution was then treated in a similar manner to that in Comparative Example 1 to prepare a liposomal dispersion.

EXAMPLE 4

DPPC, cholesterol, and $N^{\alpha}$-palmitoyl-L-arginine (hereinafter abbreviated as PAA) were placed in a test tube at a molar ratio of 1:1:0.05 to a total lipid amount of 8 μmol and dissolved in a 9:1 (by volume) mixture of chloroform and methanol. The solution was then treated in a similar manner to that in Comparative Example 1 to prepare a liposomal dispersion.

EXAMPLE 5

DPPC, cholesterol, and PAA were placed in a test tube at a molar ratio of 1:1:0.1 to a total lipid amount of 8 μmol and dissolved in a 9:1 (by volume) mixture of chloroform and methanol. The solution was then treated in a similar manner to that in Comparative Example 1 to prepare a liposomal dispersion.

EXAMPLE 6

DPPC, cholesterol, and PAA were put in a test tube at a molar ratio of 1:1:0.15 to a total lipid amount of 8 μmol and dissolved in a 9:1 (by volume) mixture of chloroform and methanol. The solution was then treated in a similar manner to that in Comparative Example 1 to prepare a liposomal dispersion.

COMPARATIVE EXAMPLE 2

In a test tube, 5.54 μmol of egg yolk lecithin, 1.85 μmol of cholesterol, and 0.62 μmol of phosphatidic acid were dissolved in a 9:1 (by volume) mixture of chloroform and methanol, and 4.0 μCi of $^{3}H$-dipalmitoylphosphatidylcholine was added to the solution. The organic solvent was then removed from the solution in a nitrogen gas stream to form a lipid film on the inner wall of the test tube. To the tube was added 5 mℓ of PBS, and the mixture was shaken in a vortex mixer and then subjected to sonication to obtain a liposomal dispersion. The dispersion was warmed to 40 to 45°C and passed through a polycarbonate membrane filter having a pore size of 0.2 μm to obtain a liposomal dispersion having a particle size of not greater than 0.2 μm.

EXAMPLE 7

In a test tube were put 4.8 μmol of egg yolk phosphatidylcholine, 1.6 μmol of cholesterol, 1.07 μmol of phosphatidic acid, and 0.53 μmol of CAEE. The lipids in the test tube were dissolved in a 9:1 (by volume) mixture of chloroform and methanol, and 2 μCi of $^{3}H$-dipalmitoylphosphatidylcholine was added thereto. The solution was then treated in a similar manner to that in Comparative Example 2 to prepare a liposomal dispersion.

EXAMPLE 8

A liposomal dispersion was prepared in a similar manner to that in Example 7, except for using 4.24 μmol

of egg yolk phosphatidylcholine, 1.41 μmol of cholesterol, 1.41 μmol of phosphatidic acid, and 0.94 μmol of CAEE.

EXAMPLE 9

In a test tube, 4.21 μmol of distearoylphosphatidylcholine, 2.11 μmol of dicetylphosphoric acid, and 1.68 μmol of CAEE were dissolved in a 9:1 (by volume) mixture of chloroform and methanol. The organic solvent was then removed in a nitrogen gas stream to form a lipid film on the inner wall of the test tube. Five milliliters of a PBS solution of 1 mM inulin containing 300 μCi of $^3$H-inulin were added to the test tube, and the mixture was treated in a similar manner to that in Comparative Example 2 to prepare a liposomal dispersion. The resulting liposomal dispersion was subjected to ultracentrifugation (150,000 × g, 1 hour, twice), and the supernatant was separated to remove the inulin unencapsulated in the liposomes. PBS was added to the residue to obtain 2.5 mℓ of a liposomal dispersion encapsulating inulin in the inner aqueous phase thereof.

As a result of enzyme assay using the choline residue of the distearoylphosphatidylcholine as a marker, it was found that the total lipid content of the resulting dispersion was 2.2 μmol per mℓ. Further, the encapsulation efficiency of inulin in the liposomes was found to be 1.8%.

TEST EXAMPLE 1

A zeta potential of each of the liposomal suspensions prepared in Comparative Example 1 and Examples 1 to 6 was measured with Zetasizer II (trademark; manufactured by Malvern Co., Ltd.). The measurement was conducted by using a capillary type cell having an inner diameter of 0.7 mm under conditions of 25°C in temperature; 0.8903 poise in solvent viscosity; 1.33 in solvent refractive index; 90 V in cell voltage; and 2 mA in cell current. The results obtained are shown in Table 1 below.

TABLE 1

ζ Potential of Liposome Suspension

| Example No. (Molar Ratio) | ζ Potential (mV) |
|---|---|
| Comparative Example 1 (DPPC:cholesterol = 1:1) | -1.97 |
| Example 1 (DPPC:cholesterol:CAEE = 1:1:0.05) | +2.72 |
| Example 2 (DPPC:cholesterol:CAEE = 1:1:0.1) | +7.81 |
| Example 3 (DPPC:cholesterol:CAEE = 1:1:0.15) | +11.2 |
| Example 4 (DPPC:cholesterol:PAA = 1:1:0.05) | -4.20 |
| Example 5 (DPPC:cholesterol:PAA = 1:1:0.1) | -4.54 |
| Example 6 (DPPC:cholesterol:PAA = 1:1:0.15) | -4.93 |

The results of Table 1 revealed that the compound of formula (I) is incorporated into the liposomal membrane.

TEST EXAMPLE 2

1) A suspension culture of MH-134 (murine hepatoma cells) (medium: RPMI-1640; pH = 7) was subjected to centrifugation (1,000 rpm, 10 mins.), and the supernatant was removed. PBS was added to the precipitate to resuspend the cells to prepare a cell suspension. Four mℓ portions of the cell suspension each containing 4 × $10^6$ cells were put in 18 test tubes and kept at 37°C.

Each of the liposomal dispersions prepared in Comparative Example 2 and Examples 7 and 8 having

been previously warmed at 37°C was added to 6 out of 18 tubes containing the cell suspension in such an amount that the total lipid content became 0.32 μmol. Three out of 6 tubes per sample were incubated for 1 hour, and the other three tubes per sample for 3.5 hours, at 37°C without shaking. Each mixture was centrifuged (1,000 rpm, 10 mins., twice in PBS) to collect the cells only. The uptake of the lipid by tumor cells was determined by measuring the radioactivity according to a liquid scintillation method. The results obtained were shown in Table 2. The numbers of cells after the incubation were corrected by quantitative determination of protein by Lowry's method.

2) Testing was carried out in the same manner as in 1) above, except that the PBS used for re-suspending tumor cells contained 5% of fetal bovine serum. The results obtained are shown in Table 3.

3) Testing was carried out in the same manner as in 1) above, except for using HL-60, human leukemia cells, in place of MH-134. The results obtained are shown in Table 4.

TABLE 2

| Incubation Time (hr) | Comparative Example 2 | Lipid Uptake by Tumor Cells, MH-134 ($nmol/4 \times 10^6$ cells), n=3 | |
|---|---|---|---|
| | | Example 7 | Example 8 |
| 1 | 1.03 ± 0.25 | 2.09 ± 0.21 | 3.53 ± 0.46 |
| 3.5 | 1.00 ± 0.11 | 2.81 ± 0.30 | 4.00 ± 0.20 |

(mean ± standard deviation)

TABLE 3

| Incubation Time (hr) | Comparative Example 2 | Lipid Uptake by Tumor Cells, MH-134 with Fetal Bovine Serum ($nmol/4 \times 10^6$ cells), n=3 | |
|---|---|---|---|
| | | Example 7 | Example 8 |
| 1 | 0.62 ± 0.09 | 1.30 ± 0.41 | 1.41 ± 0.20 |
| 3.5 | 0.73 ± 0.17 | 1.30 ± 0.02 | 2.05 ± 0.21 |

(mean ± standard deviation)

TABLE 4

| Incubation Time (hr) | Comparative Example 2 | Lipid Uptake by Tumor Cells, HL-60 (nmol/4 x $10^6$ cells), n=3 | |
|---|---|---|---|
| | | Example 7 | Example 8 |
| 1 | 0.74 ± 0.11 | 1.33 ± 0.22 | 1.13 ± 0.25 |
| 3.5 | 0.70 ± 0.08 | 1.34 ± 0.22 | 1.44 ± 0.09 |

(mean ± standard deviation)

As is apparent from Tables 2 to 4, the liposomes of the present invention were excellent in uptake by tumor cells comparing to Comparative Example 2.

Therefore, it was confirmed that the lipid membrane structures of the present invention have a specific affinity to the tumor cells and can be delivered preferentially to the tumor cells.

TEST EXAMPLE 3

The liposomal dispersion prepared in Example 9 was added to 2 mℓ of suspension culture of MH-134 cells in RPMI-1640 medium (cell number: 1.6 × $10^6$/2 mℓ) in 0.16 μmol of the total lipid content. As a control, the PBS solution of 2.62 nmol inulin containing 0.157 μCi of $^3$H-inulin was added to 2 mℓ of another suspension culture of MH-134 cells. The mixture was incubated in a similar manner to that in Test Example 2 and the sampling was carried out at 0.5, 1, 2 and 3 hours. The uptake of inulin by tumor cells was determined in a similar manner to that in Test Example 2. The numbers of cells after the incubation were corrected by quantitative determination of protein by Lowry's method. The results obtained were shown in Table 5.

TABLE 5

| Incubation Time (hr) | Inulin Uptake by Tumor Cells, MH-134 (%/1.6 x $10^6$ cells), n = 3 | |
|---|---|---|
| | Example 9 | Control (Inulin only) |
| 0.5 | 3.02 | 0.49 |
| 1 | 3.93 | 0.66 |
| 2 | 3.95 | 0.75 |
| 3 | 4.35 | 0.59 |

As is apparent from Table 5, the liposomes according to the present invention were taken up by the tumor cells, and further inulin encapsulated into the liposomes was also taken up by the tumor cells at the same time.

While the invention has been described in detail and with reference to specific embodiments thereof, it will be apparent to one skilled in the art that various changes and modifications can be made therein without departing from the spirit and scope thereof.

## Claims

1. Lipid membrane structures containing, in the lipid membrane thereof, a compound represented by formula (I):

$$R_3-(CH_2)_n\overset{\displaystyle NHR_1}{\overset{|}{C}}HCOOR_2 \qquad (I)$$

wherein $R_1$ represents a hydrogen atom or a fatty acid residue having 1 to 30 carbon atoms;
$R_2$ represents a hydrogen atom or an acyclic hydrocarbon residue having 1 to 30 carbon atoms, provided that $R_1$ and $R_2$ do not represent hydrogen atoms at the same time and that the total number of carbon atoms of $R_1$ and $R_2$ is 10 to 40;
$R_3$ represents an amino group, a guanidino group or an amidino group; and n represents an integer of from 1 to 6;
or a salt thereof, wherein the molar ratio of said compound of formula (I) or a salt thereof to the total content of the lipid components is at least about 1/40.

2. Lipid membrane structures as claimed in claim 1, wherein the fatty acid residue contains from 14 to 20 carbon atoms.

3. Lipid membrane structures as claimed in claim 1 or 2, wherein the acyclic hydrocarbon residue contains from 14 to 20 carbon atoms.

4. Lipid membrane structures as claimed in any one of claims 1 to 3, wherein n is 3 or 4.

5. Lipid membane structures as claimed in any one of claims 1 to 4, wherein $R_1$ represents a fatty acid residue, and $R_2$ represents an acyclic hydrocarbon residue, the total number of the carbon atoms of said fatty acid residue and acyclic hydrocarbon residue being 10 to 40.

6. Lipid membrane structures as claimed in any one of claims 1 to 5, wherein said membrane structures are liposomes, micelles, or microemulsions.

## Patentansprüche

1. Lipidmembranstrukturen, die in der Lipidmembran davon eine Verbindung der Formel (I)

$$R_3-(CH_2)_n\overset{\displaystyle NHR_1}{\overset{|}{C}}HCOOR_2 \qquad (I)$$

worin $R_1$ für ein Wasserstoffatom oder einen Fettsäurerest mit 1 bis 30 Kohlenstoffatomen steht; $R_2$ für ein Wasserstoffatom oder einen acyclischen Kohlenwasserstoffrest mit 1 bis 30 Kohlenstoffatomen steht, vorausgesetzt, daß $R_1$ und $R_2$ nicht gleichzeitig Waserstoffatome darstellen und daß die Gesamtzahl der Kohlenstoffatome von $R_1$ und $R_2$ 10 bis 40 ist; $R_3$ für eine Aminogruppe, eine Guanidinogruppe oder eine Amidinogruppe steht; und n für eine ganze Zahl von 1 bis 6 steht;
oder ein Salz davon enthalten, wobei das Molverhältnis der Verbindung der Formel (I) oder eines Salzes davon zu dem Gesamtgehalt der Lipidkomponenten mindestens etwa 1/40 ist.

2. Lipidmembranstrukturen nach Anspruch 1, worin der Fettsäurerest 14 bis 20 Kohlenstoffatome enthält.

3. Lipidmembranstrukturen nach Anspruch 1 oder 2, worin der acyclische Kohlenwasserstoffrest 14 bis 20 Kohlenstoffatome enthält.

4. Lipidmembranstrukturen nach einem der Ansprüche 1 bis 3, worin n für 3 oder 4 steht.

5. Lipidmembranstrukturen nach einem der Ansprüche 1 bis 4, worin $R_1$ für einen Fettsäurerest und $R_2$ für einen acyclischen Kohlenwasserstoffrest steht, wobei die Gesamtzahl der Kohlenstoffatome des Fettsäurerestes und des acyclischen Kohlenwasserstoffrestes 10 bis 40 ist.

6. Lipidmembranstrukturen nach einem der Anspüchen 1 bis 5, worin die Membranstrukturen Liposome, Micellen oder Microemulsionen sind.

## Revendications

1. Structures à membrane lipidique contenant, dans leur membrane lipidique, un composé représenté par la formule (I) :

$$R_3\text{-}(CH_2)_n CH(NHR_1)COOR_2 \qquad \text{(I)}$$

dans laquelle $R_1$ représente un atome d'hydrogène ou un reste d'acide gras en $C_1$-$C_{30}$;
$R_2$ représente un atome d'hydrogène ou un reste d'hydrocarbure acylique en $C_1$-$C_{30}$, à condition que $R_1$ et $R_2$ ne représentent pas en même temps des atomes d'hydrogène et que le nombre total d'atomes de carbone de $R_1$ et $R_2$ soit de 10 à 40;
$R_3$ représente un groupe amino, un groupe guanidino ou un groupe amidino; et n représente un entier de 1 à 6;
ou un de ses sels, dans lesquelles le rapport molaire dudit composé de formule (I) ou de son sel à la teneur totale en constituants lipidiques est d'au moins environ 1/40.

2. Structures à membrane lipidique selon la revendication 1, dans lesquelles le reste d'acide gras contient de 14 à 20 atomes de carbone.

3. Structures à membrane lipidique selon la revendication 1 ou 2 dans lesquelles le reste d'hydrocarbure acyclique contient de 14 à 20 atomes de carbone.

4. Structures à membrane lipidique selon l'une quelconque des revendications 1 à 3 dans lesquelles n est égal à 3 ou 4.

5. Structures à membrane lipidique selon l'une quelconque des revendications 1 à 4, dans lesquelles $R_1$ représente un reste d'acide gras et $R_2$ représente un reste d'hydrocarbure acyclique, le nombre total des atomes de carbone dudit reste d'acide gras et dudit reste d'hydrocarbure acyclique étant de 10 à 40.

6. Structures à membrane lipidique selon l'une quelconque des revendications 1 à 5, dans lesquelles lesdites structures à membrane sont des liposomes, des micelles ou des microémulsions.